# EUROPEAN PATENT SPECIFICATION

(11) **EP 3 367 909 B1**
(45) Date of publication and mention of the grant of the patent: **28.07.2021**
(21) Application number: 16770638.1
(22) Date of filing: 15.09.2016
(51) Int. Cl.: A61B 8/00, G10K 11/35, G10K 11/34, A61B 8/08

(54) **3D ULTRASOUND IMAGING SYSTEM FOR NERVE BLOCK APPLICATIONS**
SYSTEM ZUR 3D-ULTRASCHALLBILDGEBUNG FÜR NERVENBLOCKADEANWENDUNGEN
SYSTÈME D'IMAGERIE ULTRASONORE EN 3D POUR DES APPLICATIONS DE BLOCAGE NERVEUX

(30) Priority: 29.10.2015 US 201562247917 P
(43) Date of publication of application: 05.09.2018
(73) Proprietor: Avent, Inc., Alpharetta, GA 30004 (US)
(72) Inventor: HSU, Kenneth C., Alpharetta, Georgia 30004 (US); COKER, Justin Jeffrey, Alpharetta, Georgia 30004 (US)
(74) Representative: Dehns
(86) International application number: PCT/US2016/051890
(87) International publication number: WO 2017/074597

(56) References cited:
- EP-A1- 0 432 771
- EP-A1- 2 921 876
- EP-A2- 0 390 311
- WO-A1-2015/146588
- US-A1- 2014 180 120
- US-A1- 2015 223 777

## Description

### FIELD OF THE INVENTION

The present invention relates in general to 3D ultrasound imaging systems, and more particularly to a 3D medical ultrasound imaging system for nerve block applications.

### BACKGROUND OF THE INVENTION

In conventional ultrasound imaging, a focused beam of ultrasound energy is transmitted into body tissues to be examined and the returned echoes are detected and plotted to form an image. More specifically, some modern ultrasound systems have three-dimensional (3D) capabilities that scan a pulsed ultrasound beam in two side-wards directions relative to a beam axis. Time of flight conversion gives the image resolution along the beam direction (range), while image resolution transverse to the beam direction is obtained by the side-wards scanning of the focused beam. With such 3D imaging, a user can collect volume ultrasound data from an object and visualize any cross-section of the object through computer processing. This enables selection of the best two-dimensional (2D) image planes for a diagnosis. Even still, such 3D systems are still limited to a 2D view.

Such systems can be problematic for nerve blocks and/or various other medical procedures, since it is often desirable to locate anatomical structures and devices in a 3D space. Still additional 3D systems for addressing such limitations may include arrayed transducers, which include many ultrasound transmitters and receivers. Such transducers, however, can be expensive and bulky.

Thus, the art is continuously seeking new and improved 3D ultrasound probes. More specifically, a low cost, simplified 3D ultrasound probe that enhances the effectiveness of nerve block procedures by allowing anesthesiologists to better locate structures and/or devices would be advantageous. In addition, a 3D ultrasound probe that maintains the current transducer profile, rather than a bulky arrayed transducer, would be welcomed in the art.

Related art includes US 2014/0180120 A1 which discloses ultrasound catheters with reciprocating phased array transducers, WO 2015/146588 which discloses an ultrasonic probe, and US 2015/0223777 A1 which discloses methods and apparatuses for producing augmented images of a spine.

### BRIEF SUMMARY OF THE INVENTION

Aspects and advantages of the invention will be set forth in part in the following description, or may be obvious from the description, or may be learned through practice of the invention.

In one aspect, the present invention is directed to an ultrasound probe for imaging, the ultrasound probe being configured for placement on an outer surface of a patient's skin when generating a 3D image, as claimed in claim 1.

The ultrasound probe includes a transducer housing with a transducer transmitter configured therein. The transducer housing includes a body extending from a proximal end to a distal end along a longitudinal axis. The distal end includes an internal cavity that extends, at least, from a first side to a second side along a lateral axis of the transducer housing. The transducer transmitter is mounted to the first and second sides within the cavity. Further, the transducer transmitter is configured to rotate about the lateral axis for scanning of an ultrasound beam. Thus, during operation, the transducer transmitter is free to rotate in a clockwise direction and/or a counter-clockwise direction about the lateral axis so as to continuously scan two-dimensional (2D) images that can be used to generate a three-dimensional (3D) image.

The transducer transmitter comprises at least one plate constructed of a material configured to scan ultrasound beams, the at least one plate mounted to a shaft that is rotatable about the lateral axis, the shaft comprising a first end and a second end, the first end being mounted to the first side of the cavity of the housing and the second end being mounted to the second side of the cavity, wherein the at least one plate comprises a substantially rectangular shape.

The distal end of the body of the transducer housing comprises a lens having a linear configuration, wherein the transducer transmitter is configured adjacent to the lens. The transducer transmitter is rotatable by a motor configured within the body of the transducer housing.

It should be understood that the ultrasound probe may be configured with any of the additional features as described herein.

In another aspect, the present invention is directed to an ultrasound imaging system comprising an ultrasound probe, as claimed in claim 4.

The ultrasound imaging system includes a controller configured to receive and organize the 2D images in real-time and generate a three-dimensional (3D) image based on the 2D images.

In one embodiment, the ultrasound imaging system may also include a user interface configured to display the 3D image. More specifically, in certain embodiments, the user interface is configured to allow a user to manipulate the 3D image according to one or more user preferences.

In another embodiment, the transducer transmitter is configured to emit (or send) and/or receive ultrasound beams. More specifically, in certain embodiments, the transducer transmitter may have a gimbal configuration.

The transducer transmitter includes at least one plate mounted to a shaft that is rotatable about the lateral axis. The shaft includes a first end and a second end, with the first end being mounted to the first side of the internal cavity and the second end being mounted to the second side. In particular embodiments, the plate may be constructed of a piezoelectric material. The plate has a rectangular shape, in an embodiment a square shape.

The transducer transmitter is rotatable by a motor configured within the body of the transducer housing.

The distal end of the body of the transducer housing includes a lens having a linear configuration, wherein the transducer transmitter is configured adjacent to the lens.

In additional embodiments, the cavity of the distal end of the body of the transducer housing may extend through the proximal end of the body. In further embodiments, the distal end of the body of the transducer housing may be wider than the proximal end or vice versa. In still additional embodiments, the proximal and distal ends of the body of the housing may have substantially the same width.

In another aspect, the present disclosure is directed to a method of generating a three-dimensional (3D) ultrasound image, as claimed in claim 8. The method includes aligning an ultrasound probe at a target site of a patient on an outer surface of the patient's skin. As mentioned, the ultrasound probe includes a transducer housing with a transducer transmitter mounted therein. Further, the transducer transmitter comprises at least one plate constructed of a material configured to scan ultrasound beams, the at least one plate mounted to a shaft that is substantially parallel to the lateral axis of the housing such that the plate is rotatable about the lateral axis of the housing, wherein the at least one plate comprises a substantially rectangular shape. The distal end of the body of the transducer housing comprises a lens having a linear configuration, wherein the transducer transmitter is configured adjacent to the lens. The transducer transmitter is rotatable by a motor configured within the transducer housing.

The method also includes continuously scanning, via the transducer transmitter, two-dimensional (2D) images of the target site by rotating the transducer transmitter about the lateral axis in a clockwise direction and/or a counter-clockwise direction. Further, the method includes receiving and organizing, via a controller, the 2D images in real-time. The method also includes generating, via the controller, a three-dimensional (3D) image based on the 2D images.

The method includes displaying, via a user interface, the 3D image to a user. More specifically, in certain embodiments, the method may include allowing, via the user interface, a user to manipulate the 3D image according to one or more user preferences.

The method includes mounting the shaft within a cavity of the transducer housing such that the shaft is parallel to the lateral axis. In particular embodiments, the method may include constructing the plate from a piezoelectric material.

These and other features, aspects and advantages of the present invention will become better understood with reference to the following description and appended claims. The accompanying drawings, which are incorporated in and constitute a part of this specification, illustrate embodiments of the invention and, together with the description, serve to explain the principles of the invention.

### DESCRIPTION OF THE DRAWINGS

A full and enabling disclosure of the present invention, including the best mode thereof, directed to one of ordinary skill in the art, is set forth in the specification, which makes reference to the appended figures, in which:
FIG. 1 illustrates a schematic diagram of one embodiment of an ultrasound imaging system according to the present disclosure;
FIG. 2 illustrates a block diagram of one embodiment of suitable components that may be included in a controller of an ultrasound imaging system according to the present disclosure;
FIG. 3 illustrates a front view of one embodiment of an ultrasound probe of an ultrasound imaging system according to the present disclosure;
FIG. 4 illustrates a side view of the ultrasound probe of FIG. 3;
FIG. 5 illustrates a detailed, internal view of the distal end of the ultrasound probe of FIG. 3;
FIG. 6 illustrates another detailed, internal view of the distal end of the ultrasound probe of FIG. 3, particularly illustrating an ultrasound beam being generated by the probe for a nerve block procedure;
FIG. 7 illustrates an internal, front view of the distal end of the ultrasound probe of FIG. 5;
FIG. 8 illustrates another side view of the ultrasound probe of FIG. 3, particularly illustrating an ultrasound beam being generated by the probe for a nerve block procedure; and
FIG. 9 illustrates a flow diagram of one embodiment of a method of generating a three-dimensional (3D) ultrasound image according to the present disclosure.

### DETAILED DESCRIPTION

Reference now will be made in detail to embodiments of the invention, one or more examples of which are illustrated in the drawings. Each example is provided by way of explanation of the invention, not limitation of the invention. In fact, it will be apparent to those skilled in the art that various modifications and variations can be made in the present invention without departing from the scope of the invention. For instance, features illustrated or described as part of one embodiment can be used with another embodiment to yield a still further embodiment. Thus, it is intended that the present invention covers such modifications and variations as come within the scope of the appended claims.

Generally, the present disclosure is directed to an ultrasound imaging system having an improved ultrasound probe. For example, the ultrasound probe has a transducer housing with a transducer transmitter mounted therein. More specifically, the transducer housing has a body extending from a proximal end to a distal end along a longitudinal axis thereof. The distal end includes an internal cavity that extends, at least, from a first side to a second side along a perpendicular, lateral axis of the transducer housing. The transducer transmitter is mounted to the first and second sides within the internal cavity and is configured to rotate about the lateral axis for scanning of an ultrasound beam. Thus, during operation, the transducer transmitter is free to rotate in a clockwise direction and/or a counter-clockwise direction about the lateral axis so as to continuously scan two-dimensional (2D) images. The ultrasound imaging system may also include a controller configured to receive and organize the 2D images, e.g. in real-time, and generate a three-dimensional (3D) image based on the 2D images. Such a system can be particularly advantageous during nerve block applications as the ultrasound probe of the present disclosure can be placed at a target site of a patient (e.g. on an outer surface of the patient's skin where a nerve block procedure is to be performed at a nerve or nerve bundle therebeneath) and can remain in the same location as the probe generates the 3D image.

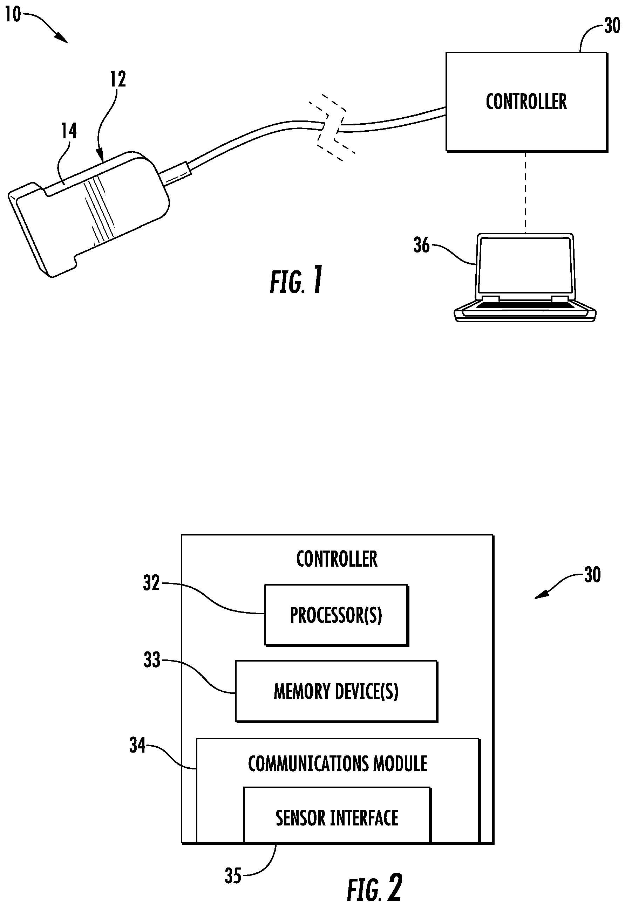

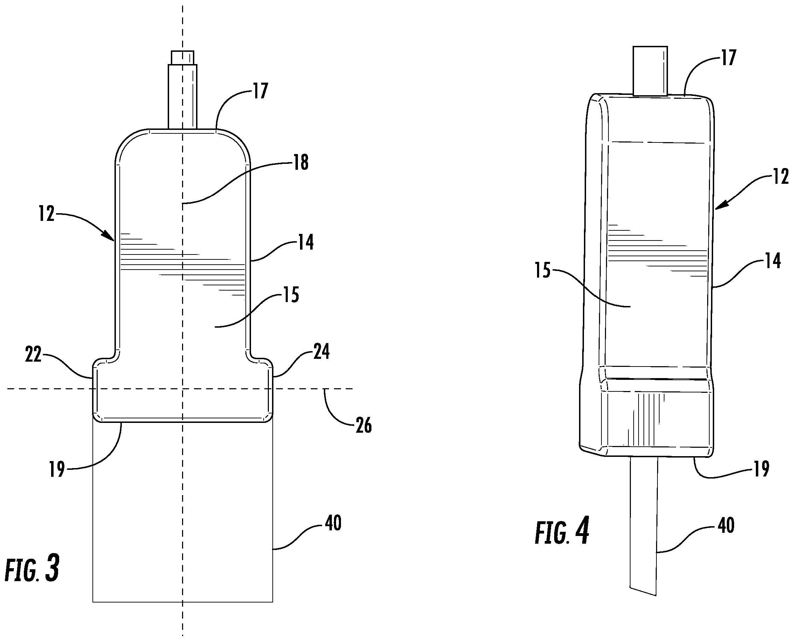

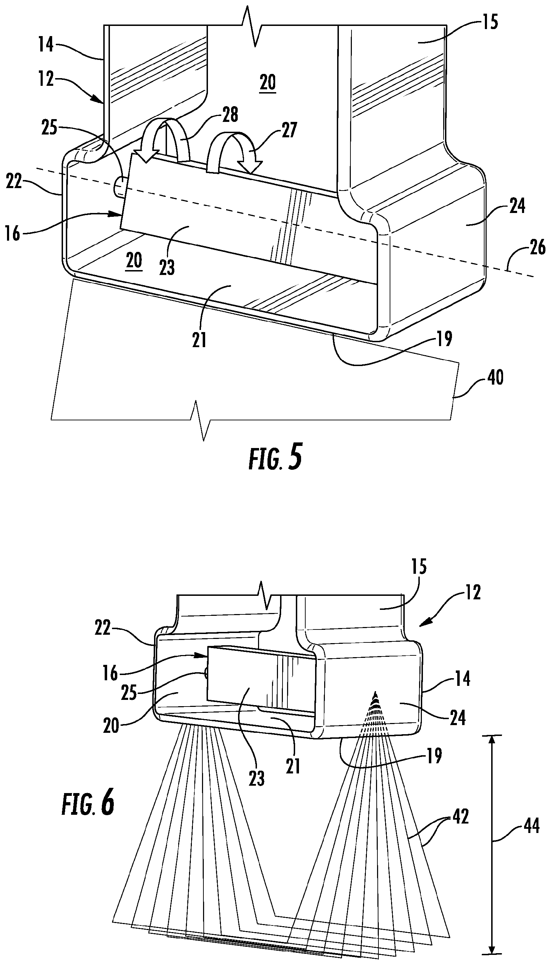

Referring now to the drawings, FIG. 1 illustrates a schematic diagram of one embodiment of an ultrasound imaging system 10 according to the present disclosure. As shown, the ultrasound imaging system 10 includes an ultrasound probe 12. More specifically, as shown in FIGS. 5 and 6, the ultrasound probe 12 has a transducer housing 14 and a transducer transmitter 16 mounted therein. Further, as shown in FIGS. 3-8, the housing 14 generally has a body 15 extending from a proximal end 17 to a distal end 19 along a longitudinal axis 18 thereof. In addition, as shown particularly in FIGS. 3 and 5-6, the distal end 19 includes an internal cavity 20 that extends, at least, from a first side 22 to a second side 24 along a lateral axis 26 of the housing 14. Further, as shown in FIG. 3, the longitudinal axis 18 is generally perpendicular to the lateral axis 26.

In additional embodiments, as shown in FIGS. 5 and 6, the internal cavity 20 at the distal end 19 of the body 15 of the transducer housing 14 may extend through the proximal end 17 of the body 15. In other words, as shown in FIGS. 5-7, the internal cavity 20 may encompass substantially the entire housing 14. In addition, as shown generally in the figures, the distal end 19 of the body 15 of the housing 14 may be wider than the proximal end 17 of the body 15, e.g. such that the proximal end 17 of the body 15 can be easily gripped by a user. Alternatively, the distal end 19 of the body 15 of the housing 14 may be narrower than the proximal end 17 of the body 15. In still another embodiment, the proximal and distal ends 17, 19 of the body 15 of the housing 14 have substantially the same width along the longitudinal axis 18.

In addition, as shown in FIGS. 5 and 6, the distal end 19 of the body 15 of the transducer housing 14 may also include a lens 21 having any suitable configuration. Thus, the lens 21 is configured to allow passage of the ultrasonic beams 42 therethrough. As shown, the lens 21 has a linear configuration. In alternative arrangements not within the scope of the appended claims, the lens 21 may have a convex configuration. Thus, as shown, the transducer transmitter 16 may be configured adjacent to the lens 21.

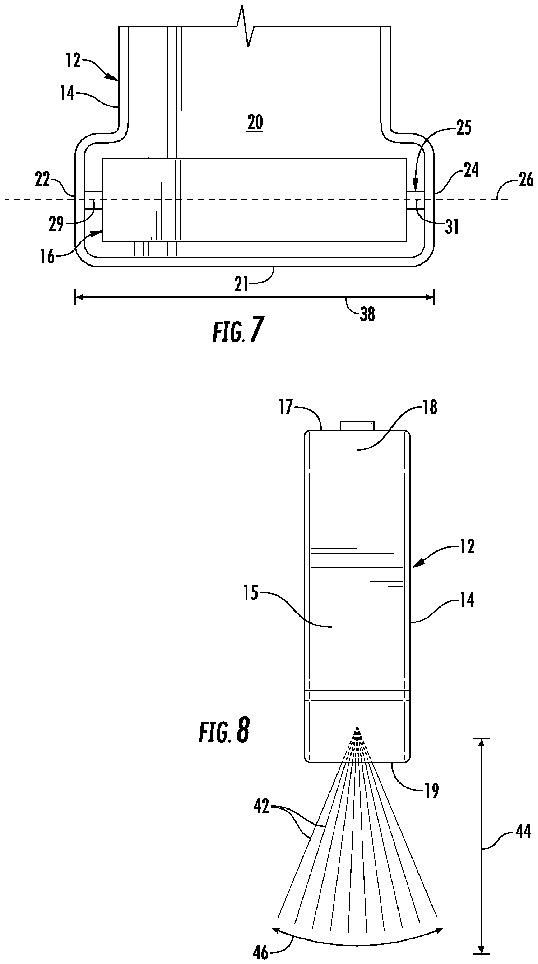

As is generally understood, the transducer transmitter 16 is configured to emit and/or receive ultrasound beams. For example, as shown in FIGS. 5 and 6, the transducer transmitter 16 may be mounted to the first and second sides 22, 24 of the internal cavity 20 such that the transmitter 16 is configured to rotate about the lateral axis 26 for scanning ultrasound beams. More specifically, in certain embodiments, the transducer transmitter 16 may have a gimbal configuration. As used herein, a "gimbal configuration" generally refers to a pivoted support that allows for rotation of an object about a single axis. Thus, as shown in FIGS. 5 and 6, the transducer transmitter 16 includes at least one plate 23 mounted to a shaft 25 that is rotatable about the lateral axis 26. Further, as shown in FIG. 7, the shaft 25 includes a first end 29 and a second end 31. More specifically, as shown, the first end 29 of the shaft 25 is mounted to the first side 22 of the internal cavity 20 of the transducer housing 14, whereas the second end 31 is mounted to the opposing, second side 24 of the internal cavity 20. As such, the plate 23 can be mounted along any portion of the length 38 of the shaft 25. For example, as shown, the plate 23 extends substantially the length 38 of the shaft 25. In addition, as shown, the plate 23 may have a solid configuration (as shown) or may have a segmented configuration.

It should be understood that the plate 23 is constructed of any suitable material configured to scan ultrasound beams. For example, in particular embodiments, the plate 23 may be constructed of a piezoelectric material. As shown, the plate 23 has a rectangular shape. In an embodiment, the plate 23 may have a square shape.

Thus, during operation, the probe 12 can be placed at a target site of the patient and while maintaining the probe 12 in its initial position, the plate 23 of the transducer transmitter 16 is free to rotate about the shaft 25 in a clockwise direction (as indicated by arrow 27 in FIG. 5) and/or a counter-clockwise direction (as indicated by arrow 28 in FIG. 5) about the lateral axis 26 so as to continuously scan two-dimensional (2D) images in an ultrasound plane 40, e.g. by generating multiple ultrasound beams 42 (FIGS. 6 and 8). More specifically, the transducer transmitter 16 is rotated by a motor configured within the body 15 of the transducer housing 14. As such, the probe 12 can be particularly advantageous for nerve block applications as the plate 23 is configured to generate particularly useful images at a predetermined depth 44, which corresponds to a location of a nerve or nerve bundle. Further, as shown in FIG. 8, the width 46 of the image may be adjusted based on a variety of design factors. For example, the width 46 of the image can be modified by changing the dimensions of the plate 23 (e.g. length, width, height, etc.), the speed of rotation of the shaft 25, the angle of the plate 23 with respect to the shaft 25, or similar.

Referring back to FIGS. 1 and 2, the ultrasound imaging system 10 may also include a controller 30 configured to receive and organize the 2D images generated by the transducer transmitter 16 in real-time and generate a three-dimensional (3D) image based on the 2D images. More specifically, as shown in FIG. 2, there is illustrated a block diagram of one embodiment of suitable components that may be included within the controller 30 in accordance with aspects of the present subject matter. As shown, the controller 30 may include one or more processor(s) 32 and associated memory device(s) 33 configured to perform a variety of computer-implemented functions (e.g., performing the methods, steps, and the like and storing relevant data as disclosed herein). Additionally, the controller 30 may also include a communications module 34 to facilitate communications between the controller 30 and the various components of the system 10. Further, the communications module 34 may include a sensor interface 35 (e.g., one or more analog-to-digital converters) to permit signals transmitted from the probe 12 to be converted into signals that can be understood and processed by the processors 32. In addition, as shown, the ultrasound imaging system 10 may also include a user interface 36 (FIG. 1) configured to display the 3D image. More specifically, in certain embodiments, the user interface 36 may be configured to allow a user to manipulate the 3D image according to one or more user preferences.

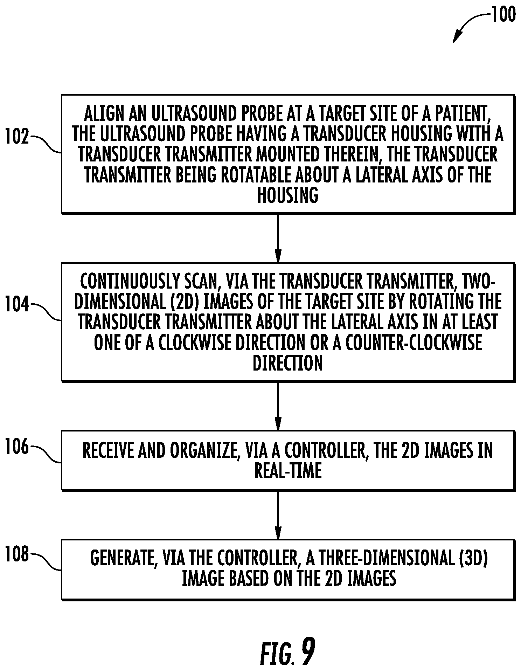

Referring now to FIG. 9, a flow diagram of one embodiment of a method 100 of generating a three-dimensional (3D) ultrasound image is illustrated. As shown at 102, the method 100 includes aligning an ultrasound probe 12 at a target site of a patient. For example, the probe 12 may be aligned at a location that corresponds to a nerve or nerve bundle where a nerve block procedure is to be performed. As mentioned, the ultrasound probe 12 includes a transducer housing 14 with a transducer transmitter 16 mounted therein. Further, the transducer transmitter 16 is configured to rotate about the lateral axis 26 of the housing 14. Thus, as shown at 104, the method 100 includes continuously scanning, via the transducer transmitter 16, two-dimensional (2D) images (e.g. as indicated by ultrasonic beams 42) of the target site by rotating the transducer transmitter 16 about the lateral axis 26 in a clockwise direction 27 and/or a counter-clockwise direction 28. As shown at 106, the method 100 includes receiving and organizing, via a controller, the 2D images in real-time. As shown at 108, the method 100 includes generating, via the controller, a three-dimensional (3D) image based on the 2D images.

In addition, in one embodiment, the method 100 may also include displaying, via a user interface 36, the 3D image to a user. More specifically, in certain embodiments, the method 100 may include allowing, via the user interface 36, a user to manipulate the 3D image according to one or more user preferences.

As mentioned in reference to FIG. 4, the transducer transmitter 16 includes at least one plate 23 mounted to a shaft 25 that is rotatable about the lateral axis 26. Thus, the method 100 includes mounting the shaft 26 within the internal cavity 20 of the transducer housing 14 such that the shaft 25 is parallel to the lateral axis 26. The method 100 includes rotating the transducer transmitter 16 by a motor configured within the transducer housing 14 (not shown). Accordingly, when the probe 12 is located at a target site of the patient, the transducer transmitter 16 is configured to continuously rotate so as to generate a 3D image of an object at a depth 44. Further, it should be understood that the ultrasound imaging system 10 may include any of the additional features as described herein.

While the disclosure has been described in detail with respect to specific embodiments thereof, it will be apparent to those skilled in the art that various alterations, modifications and other changes may be made to the disclosure without departing from the scope of the invention, as set out in the appended claims.

## Claims

1. An ultrasound probe (12) for imaging, the ultrasound probe being configured for placement on an outer surface of a patient's skin when generating a 3D image, the ultrasound probe comprising:
a transducer housing (14) comprising a body (15) extending from a proximal end (17) to a distal end (19) along a longitudinal axis (18), the distal end (19) comprising an internal cavity (20) that extends, at least, from a first side (22) to a second side (24) along a lateral axis (26) of the transducer housing; and **characterised by** comprising
a transducer transmitter (16) mounted to the first and second sides within the cavity, the transducer transmitter (16) configured to emit and receive ultrasound beams, the transducer transmitter (16) being rotatable about the lateral axis (26) for scanning of an ultrasound beam, wherein, during operation, the transducer transmitter is free to rotate in a clockwise direction and a counter-clockwise direction about the lateral axis so as to continuously scan two-dimensional (2D) images that can be used to generate a three-dimensional (3D) image;
wherein the transducer transmitter (16) comprises at least one plate (23) constructed of a material configured to scan ultrasound beams, the at least one plate (23) mounted to a shaft (25) that is rotatable about the lateral axis, the shaft comprising a first end (29) and a second end (31), the first end (29) being mounted to the first side (22) of the cavity of the housing and the second end (31) being mounted to the second side (24) of the cavity, wherein the at least one plate (23) comprises a rectangular shape;
wherein the distal end (19) of the body (15) of the transducer housing (14) comprises a lens (21) having a linear configuration, and wherein the transducer transmitter (16) is configured adjacent to the lens (21); and
wherein the transducer transmitter (16) is rotatable by a motor configured within the body (15) of the transducer housing (14).

2. The ultrasound probe of claim 1, wherein the transducer transmitter (16) comprises a gimbal configuration.

3. The ultrasound probe of claim 1 or 2 wherein the at least one plate (23) is constructed of a piezoelectric material.

4. An ultrasound imaging system (10), comprising:
the ultrasound probe (12) of any preceding claim; and
a controller configured to receive and organize the 2D images in real-time and generate a three-dimensional (3D) image based on the 2D images.

5. The ultrasound imaging system of claim 4, further comprising a user interface configured to display the 3D image, the user interface configured to allow a user to manipulate the 3D image according to one or more user preferences.

6. The ultrasound imaging system of claim 4 or 5, wherein the cavity of the distal end (19) of the body (15) of the transducer housing (14) extends through the proximal end (17) of the body (15).

7. The ultrasound imaging system of any of claims 4 to 6, wherein the distal end (19) of the body (15) of the transducer housing (14) is wider than the proximal end (17).

8. A method of generating a three-dimensional ultrasound image, the method comprising:
aligning an ultrasound probe (12) at a target site of a patient on an outer surface of the patient's skin, the ultrasound probe (12) having a transducer housing (14) with a transducer transmitter (16) mounted therein, wherein:
the transducer transmitter (16) comprises at least one plate (23) constructed of a material configured to scan ultrasound beams, the at least one plate (23) mounted to a shaft (25) that is parallel to a lateral axis (26) of the housing such that the plate (23) is rotatable about the lateral axis (26), wherein the at least one plate (23) comprises a rectangular shape; wherein the distal end (19) of the body (15) of the transducer housing (14) comprises a lens (21) having a linear configuration, and wherein the transducer transmitter (16) is configured adjacent to the lens (21); and
the transducer transmitter (16) is rotatable by a motor configured within the transducer housing (14);
continuously scanning, via the transducer transmitter (16), two-dimensional (2D) images of the target site by rotating the transducer transmitter (16) about the lateral axis (26) in at least one of a clockwise direction or a counter-clockwise direction;
receiving and organizing, via a controller, the 2D images in real-time;
generating, via the controller, a three-dimensional (3D) image based on the 2D images; and
displaying, via a user interface, the 3D image to a user.

9. The method of claim 8, further comprising allowing, via the user interface, a user to manipulate the 3D image according to one or more user preferences.

## Patentansprüche

1. Ultraschallsonde (12) zur Bildgebung, wobei der Ultraschallsonde für die Platzierung an einer Außenfläche der Haut eines Patienten konfiguriert ist, wenn ein 3D-Bild erzeugt wird, wobei der Ultraschallsonde Folgendes umfasst:
ein Schallkopfgehäuse (14), umfassend einen Körper (15), der sich entlang einer Längsachse (18) von einem proximalen Ende (17) zu einem distalen Ende (19) erstreckt, wobei das distale Ende (19) einen inneren Hohlraum (20) umfasst, der sich entlang einer Seitenachse (26) des Schallkopfgehäuses mindestens von einer ersten Seite (22) zu einer zweiten Seite (24) erstreckt; und **dadurch gekennzeichnet, dass** es Folgendes umfasst
einen Schallkopfsender (16), der an der ersten und zweiten Seite innerhalb des Hohlraums montiert ist, wobei der Schallkopfsender (16) konfiguriert ist, um Ultraschallstrahlen abzugeben und zu empfangen, wobei der Schallkopfsender (16) zum Scannen eines Ultraschallstrahls um die Seitenachse (26) drehbar ist, wobei während des Betriebs der Schallkopfsender in eine Richtung im Uhrzeigersinn und entgegen des Uhrzeigersinns um die Seitenachse frei drehen kann, um kontinuierlich zweidimensionale (2D) Bilder zu scannen, die verwendet werden können, um ein dreidimensionales (3D) Bild zu erzeugen;
wobei der Schallkopfsender (16) mindestens eine Platte (23) umfasst, die aus einem Material konstruiert ist, das zum Scannen von Ultraschallstrahlen konfiguriert ist, wobei die mindestens eine Platte (23) auf einer Welle (25) montiert ist, die um die Seitenachse drehbar ist, wobei die Welle ein erstes Ende (29) und ein zweites Ende (31) umfasst, wobei das erste Ende (29) an der ersten Seite (22) des Hohlraums des Gehäuses montiert ist und das zweite Ende (31) an der zweiten Seite (24) des Hohlraums montiert ist, wobei die mindestens eine Platte (23) eine rechteckige Form umfasst;
wobei das distale Ende (19) des Körpers (15) des Schallkopfgehäuses (14) eine Linse (21) umfasst, die eine lineare Konfiguration aufweist, und wobei der Schallkopfsender (16) angrenzend zu der Linse (21) konfiguriert ist; und
wobei der Schallkopfsender (16) innerhalb des Körpers (15) des Schallkopfgehäuses (14) durch einen Motor drehbar konfiguriert ist.

2. Ultraschallsonde nach Anspruch 1, wobei der Schallkopfsender (16) eine kardanisch aufgehängte Konfiguration umfasst.

3. Ultraschallsonde nach Anspruch 1 oder 2, wobei die mindestens eine Platte (23) aus einem piezoelektrischen Material konstruiert ist.

4. System zur Ultraschall-Bildgebung (10), umfassend:
die Ultraschallsonde (12) nach einem vorstehenden Anspruch; und
eine Steuereinheit, die konfiguriert ist, um die 2D-Bilder in Echtzeit zu empfangen und zu organisieren und auf der Grundlage der 2D-Bilder ein dreidimensionales (3D) Bild zu erzeugen.

5. System zur Ultraschall-Bildgebung nach Anspruch 4, weiter umfassend eine Benutzeroberfläche, die konfiguriert ist, um das 3D-Bild anzuzeigen, wobei die Benutzeroberfläche konfiguriert ist, um es einem Benutzer zu erlauben, das 3D-Bild gemäß einem oder mehreren Benutzervorzügen zu manipulieren.

6. System zur Ultraschall-Bildgebung nach Anspruch 4 oder 5, wobei sich der Hohlraum des distalen Endes (19) des Körpers (15) des Schallkopfgehäuses (14) durch das proximale Ende (17) des Körpers (15) erstreckt.

7. System zur Ultraschall-Bildgebung nach einem der Ansprüche 4 bis 6, wobei das distale Ende (19) des Körpers (15) des Schallkopfgehäuses (14) breiter ist als das proximale Ende (17).

8. Verfahren zum Erzeugen eines dreidimensionalen Ultraschallbilds, wobei das Verfahren Folgendes umfasst:
Ausrichten einer Ultraschallsonde (12) auf einem Zielbereich eines Patienten an einer Außenfläche der Haut des Patienten, wobei die Ultraschallsonde (12) ein Schallkopfgehäuse (14) mit einem darin montierten Schallkopfsender (16) aufweist, wobei:
der Schallkopfsender (16) mindestens eine Platte (23) umfasst, die aus einem Material konstruiert ist, das zum Scannen von Ultraschallstrahlen konfiguriert ist, wobei die mindestens eine Platte (23) auf einer Welle (25) montiert ist, die parallel zu einer Seitenachse (26) des Gehäuses ist, sodass die Platte (23) um die Seitenachse (26) drehbar ist, wobei die mindestens eine Platte (23) eine rechteckige Form umfasst; wobei das distale Ende (19) des Körpers (15) des Schallkopfgehäuses (14) eine Linse (21) umfasst, die eine lineare Konfiguration aufweist, und wobei der Schallkopfsender (16) angrenzend zu der Linse (21) konfiguriert ist; und
der Schallkopfsender (16) innerhalb des Körpers (15) des Schallkopfgehäuses (14) durch einen Motor drehbar konfiguriert ist;
kontinuierliches Scannen von zweidimensionalen (2D) Bildern des Zielbereichs über den Schallkopfsender (16) durch Drehen des Schallkopfsenders (16) um die Seitenachse (26) in mindestens eines von einer Richtung im Uhrzeigersinn oder einer Richtung gegen den Uhrzeigersinn;
Empfangen und Organisieren der 2D-Bilder in Echtzeit über eine Steuereinheit;
Erzeugen eines dreidimensionalen (3D) Bilds auf der Grundlage der 2D-Bilder über eine Steuerung; und
Anzeigen des 3D-Bilds einem Benutzer über eine Benutzeroberfläche.

9. Verfahren nach Anspruch 8, weiter umfassend, es einem Benutzer zu erlauben, über die Benutzeroberfläche das 3D-Bild gemäß einem oder mehreren Benutzervorzügen zu manipulieren.

## Revendications

1. Sonde ultrasonore (12) destinée à l'imagerie, la sonde ultrasonore étant configurée pour être placée sur une surface externe de la peau d'un patient lors de la génération d'une image 3D, la sonde ultrasonore comprenant :
un boîtier de transducteur (14) comprenant un corps (15) s'étendant d'une extrémité proximale (17) à une extrémité distale (19) le long d'un axe longitudinal (18), l'extrémité distale (19) comprenant une cavité interne (20) qui s'étend, au moins, d'un premier côté (22) à un second côté (24) le long d'un axe latéral (26) du boîtier de transducteur ; et
**caractérisée en ce qu'**elle comprend
un transducteur-émetteur (16) monté sur les premier et second côtés au sein de la cavité, le transducteur-émetteur (16) étant configuré pour émettre et recevoir des faisceaux ultrasonores, le transducteur-émetteur (16) pouvant tourner autour de l'axe latéral (26) pour balayer un faisceau ultrasonore, dans laquelle, en fonctionnement, le transducteur-émetteur est libre de tourner dans le sens des aiguilles d'une montre et dans le sens inverse des aiguilles d'une montre autour de l'axe latéral afin de balayer en continu des images en deux dimensions (2D) qui peuvent être utilisées pour générer une image en trois dimensions (3D) ;
dans laquelle le transducteur-émetteur (16) comprend au moins une plaque (23) composée d'un matériau configuré pour balayer des faisceaux ultrasonores, l'au moins une plaque (23) étant montée sur un arbre (25) qui peut tourner autour de l'axe latéral, l'arbre comprenant une première extrémité (29) et une seconde extrémité (31), la première extrémité (29) étant montée sur le premier côté (22) de la cavité du boîtier et la seconde extrémité (31) étant montée sur le second côté (24) de la cavité, dans laquelle l'au moins une plaque (23) comprend une forme rectangulaire
dans laquelle l'extrémité distale (19) du corps (15) du boîtier de transducteur (14) comprend une lentille (21) présentant une configuration linéaire, et dans laquelle le transducteur-émetteur (16) est configuré de manière adjacente à la lentille (21) ; et
dans laquelle le transducteur-émetteur (16) peut tourner grâce à un moteur configuré au sein du corps (15) du boîtier de transducteur (14).

2. Sonde ultrasonore selon la revendication 1, dans laquelle le transducteur-émetteur (16) comprend une configuration de cardan.

3. Sonde ultrasonore selon la revendication 1 ou 2, dans laquelle l'au moins une plaque (23) est composée d'un matériau piézoélectrique.

4. Système d'imagerie ultrasonore (10), comprenant :
la sonde ultrasonore (12) selon une quelconque revendication précédente ; et
un dispositif de commande configuré pour recevoir et organiser les images 2D en temps réel et générer une image en trois dimensions (3D) sur la base des images 2D.

5. Système d'imagerie ultrasonore selon la revendication 4, comprenant en outre une interface utilisateur configurée pour afficher l'image 3D, l'interface utilisateur étant configurée pour permettre à un utilisateur de manipuler l'image 3D selon une ou plusieurs préférences utilisateur.

6. Système d'imagerie ultrasonore selon la revendication 4 ou 5, dans lequel la cavité de l'extrémité distale (19) du corps (15) du boîtier de transducteur (14) s'étend à travers l'extrémité proximale (17) du corps (15).

7. Système d'imagerie ultrasonore selon l'une quelconque des revendications 4 à 6, dans lequel l'extrémité distale (19) du corps (15) du boîtier de transducteur (14) est plus grande que l'extrémité proximale (17).

8. Procédé de génération d'une image ultrasonore en trois dimensions, le procédé comprenant :
l'alignement d'une sonde ultrasonore (12) au niveau d'un site cible d'un patient sur une surface externe de la peau du patient, la sonde ultrasonore (12) présentant un boîtier de transducteur (14) avec un transducteur-émetteur (16) monté dans celui-ci, dans lequel :
le transducteur-émetteur (16) comprend au moins une plaque (23) composée d'un matériau configuré pour balayer des faisceaux ultrasonores, l'au moins une plaque (23) étant montée sur un arbre (25) qui est parallèle à un axe latéral (26) du boîtier de sorte que la plaque (23) puisse tourner autour de l'axe latéral (26), dans lequel l'au moins une plaque (23) comprend une forme rectangulaire; dans lequel l'extrémité distale (19) du corps (15) du boîtier de transducteur (14) comprend une lentille (21) présentant une configuration linéaire, et dans lequel le transducteur-émetteur (16) est configuré de manière adjacente à la lentille (21) ; et
le transducteur-émetteur (16) peut tourner grâce à un moteur configuré au sein du boîtier de transducteur (14) ;
le balayage continu, par l'intermédiaire du transducteur-émetteur (16), d'images en deux dimensions (2D) du site cible par rotation du transducteur-émetteur (16) autour de l'axe latéral (26) dans au moins l'un d'un sens des aiguilles d'une montre ou d'un sens inverse des aiguilles d'une montre ;
la réception et l'organisation, par l'intermédiaire d'un dispositif de commande, des images 2D en temps réel ;
la génération, par l'intermédiaire du dispositif de commande, d'une image en trois dimensions (3D) sur la base des images 2D ; et
l'affichage, par l'intermédiaire d'une interface utilisateur, de l'image 3D à l'attention d'un utilisateur.

9. Procédé selon la revendication 8, comprenant en outre le fait de permettre, par l'intermédiaire de l'interface utilisateur, à un utilisateur de manipuler l'image 3D selon une ou plusieurs préférences utilisateur.
